# EUROPEAN PATENT APPLICATION

(11) **EP 2 450 058 A1**
(43) Date of publication of application: **09.05.2012**
(21) Application number: 10398005.8
(22) Date of filing: 23.08.2010
(51) Int. Cl.: A61K 47/48

(54) **Antimicrobial nanoparticle conjugates**

(71) Applicant: Matera Lda, Cantanhede 3060-197 (PT)
(72) Inventor: de Silva Ferreira, Lino, 3030-076 Coimbra (PT); da Silva Oliveira Paulo, Cristiana, 3060-125 Cantanhede (PT)
(74) Representative: Sampson, Eimear

(57) **Abstract**

The present invention provides antimicrobial nanoparticle conjugates and an efficient method of preparing same. In particular, the invention relates to the preparation of antifungal nanoparticle conjugates comprising amphotericin B covalently immobilized to nanoparticles. The conjugates, and suspensions thereof, can be used to form antifungal coatings with particular application to medical devices and materials.

## Description

The present invention relates to antimicrobial nanoparticle conjugates and methods of their preparation and, in particular to antifungal nanoparticle conjugates comprising amphotericin B covalently immobilized to silica nanoparticles. Conjugates and suspensions thereof can be used to form antifungal coatings with particular application to medical devices and materials.

Since their discovery in the 20^{th} century, antimicrobial agents have substantially mitigated the threat posed by infectious diseases. However, infections caused by bacteria, fungi, parasites and viruses remain prevalent, and are of increasing concern in hospital settings where the incidence of infection is increasing rapidly. In 2002, an estimated 1.7 million people in the United States of America alone were infected with microorganisms during a stay in hospital, with 99,000 resulting deaths reported (Klevins et al., Public Health Rep, 2007, 122, 160-166).

The rise in reported nosocomial infections can be attributed in part to increased resistance of microbes to drugs, as well as to large numbers of elderly or immunocompromised patients requiring hospital treatment. In addition, increased use of invasive devices such as intubation tubes and catheters allow microbes to bypass the body's natural defence mechanisms and provide direct routes for microbial infection. Poor hospital hygiene practices have also been indicated as significantly causative in the rise of microbial infections, with both direct and indirect contact transmission leading to infection. In particular, indirect-contact transmission resulting from the transfer of microbes from contaminated surfaces to susceptible patients has been implicated in a large percentage of nosocomial cases. Such transmission typically involves the transfer of microbes from unhygienic surfaces of medical instruments and materials, resulting in infection. Of these cases, fungal infections are estimated to account for one in four reported incidences. (Wisplinghoff et al., Clin Infect Dis. 2004, 39, 309).

Polyene macrolides are known for their antifungal activity, and this class includes clinically significant drugs such as amphotericin B (AmB), nystatin and natamycin. These drugs are characterised by a hydroxylated macrocyclic lactone ring of amphipathic nature, typically containing a single sugar. A chromophore formed by a system of three to seven conjugated double bonds in the macrolactone ring gives rise to characteristic physicochemical properties for this class of drugs, including strong UV-VIS light absorption and poor water solubility (Aparicio, J. Chemistry and Biology 12, May 2005).

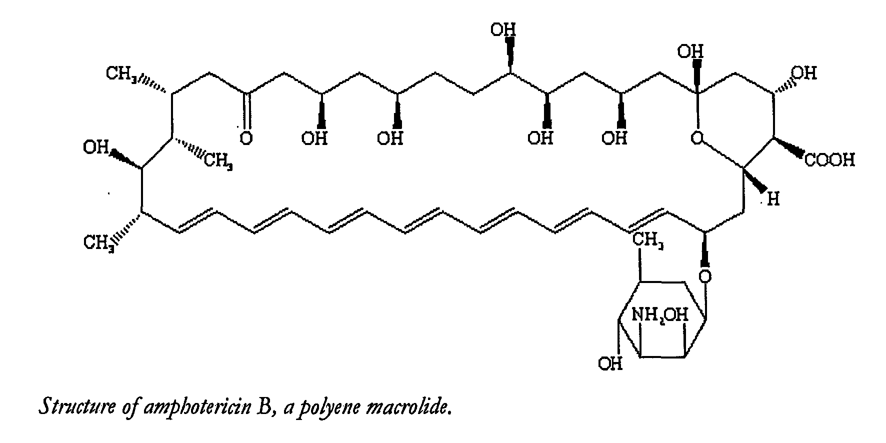

Although the mode of action of these macrolytic antifungals has not been fully elucidated, it is generally accepted that preferential binding of the drug to cytoplasmic membrane sterols, causes changes in the permeability of the cell membrane. This disruption causes the contents of the cell to 'leak', resulting ultimately in fungal cell death.

Of the polyene antifungals, amphotericin B is a commonly-used antifungal agent, which has played a major role in the treatment and management of systemic fungal infections, since its antifungal activity was first demonstrated in the 1950s. Amphotericin B demonstrates both fungistatic and fungicidal activity and has proven to be effective against a wide variety of fungal species. The broad spectrum of antifungal activity of amphotericin B includes most of the medically significant fungi including yeasts (*Candida albicans, Candida neoformans*), endemic mycoses (*Histoblasma capsulatum, Blastomyces dermatitidis and Coccidioides immitis)* and molds *(Aspergillus fumigatus, Mucor).* Few fungal strains resistant to amphotericin B have been reported, making it the drug of choice in the therapeutic treatment of many systemic fungal infections. Efficacious drugs are therefore available for therapeutic treatment of microbial infections. However, it would be beneficial to exploit the significant antimicrobial activity of agents such as amphotericin B, to address the problems of indirect-contact transmission of microbes, by incorporating them into materials or coatings which can prevent microbial colonization or biofilm formation. However, difficulties associated with incorporating such agents into suitable vehicles for delivery or controlled release of the active agent, concerns about systemic toxicity, and the limited life-spans of antimicrobial-incorporating formulations, have thus far all limited the usefulness of amphotericin B and other antimicrobial agents for such applications.

Incorporation of antimicrobial agents into controlled-release formulations has been described for application to systemic fungal infections. Marra *et al.* have described the incorporation of amphotericin B into bone cements for the treatment of osteomyelitis caused by *Candida albicans* (Marra et al., Can J Surg 2001, 44(5), 383-386). Amphotericin B in a sterile powder dosage form was added to polymethylmethacrylate bone cement to create an antifungal-loaded cement which was used in the treatment of an adult who had undergone multiple revisions of a hip prosthesis. The amphotericin B was released by diffusion of the drug into blood and local wound fluid, and the highest wound drainage drug concentrations were measured for 2 days post-operatively, before dropping sharply.

Amphotericin B has also been encapsulated in microsphere formulations (Angra et al., J Microencapsul 2009, 26, 580; Nahar et al., nanomedicine 2008, 4(3), 252-261) for controlled therapeutic delivery. In these reports, entrapment of amphotericin B into nanoparticles of different gelatins (Nahar *et al*) and into microspheres in cross-linked bovine serum albumin (Angra *et al.*) was studied in order to evaluate drug release and toxicity.

However, these controlled-release formulations were found to release amphotericin B primarily by diffusion, exhibiting biphasic release characterised by an initial burst of release of the active agent followed by slow and limited release. Consequently, these formulations have limited life-spans, with the majority of amphotericin B being released within a matter of days. Therefore, these formulations are inefficient for use in materials or coatings intended for reusable devices or materials, and for which a longer antimicrobial effect would be highly desirable. In addition, large concentrations of the active agent are also required for encapsulation in these formulations, making them unsuitable for application to certain materials and devices, which may be structurally impaired by the high drug loading.

Gel formulations incorporating amphotericin B have recently been proposed as antifungal preparations. These encapsulated formulations allow slow diffusion of amphotericin B from the gel matrix over time, increasing the life-span of the antifungal activity. Hudson *et al* (Hudson et al., Biomaterials 2009, 31, 1444-1452) describe an antifungal gel formulation for treating localised infections, comprising cross-linked gels containing amphotericin B conjugated to the hydrogel or suspended in the gel matrix. The gel provided *in vitro* release of antifungal activity for 11 days, while direct contact with the gel killed *Candida albicans* for three weeks

Zumbuehl *et al.* describe dextran-based hydrogels into which amphotericin B is adsorbed. The gels killed fungi within 2 hours of contact and maintained antifungal activity against *Candida albicans* for 53 days.

However, despite the increase in length of antifungal activity demonstrated for these gel formulations, life-spans are still often unacceptably short for application to reusable medical devices and materials. Thus, while gel formulations show some promise for therapeutic applications, they are of limited use in the preparation and development of coatings for medical devices and materials. In addition, the low mechanical properties of the gels and the requirement to modify substrate surfaces to create gel-anchorage points make these formulations of limited practical application to a wide spectrum of medical devices and materials.

As described above, high loadings of antimicrobial agents are often required for controlled release formulations, prohibiting their use for certain applications. In addition, slow release of active agents in therapy may cause regions of sublethal drug concentrations to develop at the release site, leading to drug resistance. Thus, more recent strategies have focused on the development of formulations that do not release the antifungal agent through diffusion. Wu *et al.* describes the use of amphotericin B-functionalized carbon nanotubes for delivering therapeutic molecules into cells for the treatment of chronic fungal infections. However, doubts remain about the viability of using carbon nanotubes for coating materials, due to cytotoxicity concerns, the expense associated with modification of large surfaces, and the difficulties in processing large amounts of bioactive agents.

Therefore, despite active research in this area, there remains a need for novel antimicrobial preparations which can be used to create coatings which exhibit antimicrobial properties and can readily be applied to medical devices and materials to reduce or prevent antimicrobial colonization or biofilm formation. In addition, it would be desirable to provide antimicrobial coatings or materials which expose the microbes to high surface concentrations of the antimicrobial agent, and which exhibit sufficient life-spans to make them viable for use on medical devices and materials.

The applicant has now discovered that antimicrobial agents can be immobilized to nanoparticles by linker molecules, to form antimicrobial nanoparticle conjugates, which address some of the disadvantages of the prior art formulations.

Accordingly, in an aspect of the invention there is provided an antimicrobial nanoparticle conjugate comprising an antimicrobial agent immobilized to a nanoparticle by a linker molecule.

The term "antimicrobial agent" as used herein is taken to mean a substance that kills (microbicidal) or inhibits (microbistatic) the growth of microorganisms. The term "antimicrobial agent" thus encompasses antibiotics, antivirals, antifungals and antiparasitics.

The term "immobilized" as used herein is taken to mean coupling of the antimicrobial agent and the nanoparticle such that substantial leaching of the antimicrobial agent does not occur. Leaching can be evaluated by the activity of the antimicrobial agent present in an incubation medium after a defined period of time.

"Substantial leaching" can be defined as the presence of sufficient antimicrobial agent in an incubation medium after 8 hours of incubation to kill more than 50% of the initial number of microbes. In practice, for example, "sufficient leaching" of an antifungal agent can be determined by incubating the conjugate (5 mg), or a substrate (1 cm²) or device (1 cm²) coated with the conjugate, with 1 ml Yeast Extract Peptone Dextrose (YPD) medium containing 1×10⁵ yeast cells for 8 h, at 30°C and 150 rpm orbital shaking. An aliquot of the medium is then serially diluted with sterile water and plated on YPD agar. The plates are incubated at 30°C for 24 h and the number of Colony Forming Units (CFU) are counted and compared against a control, in which *Candida* is incubated without the material, substrate or device).

The term "nanoparticle" as used herein is taken to mean particles having one or more dimensions at the nanometre scale and preferably those particles having one or more dimensions of 1 to 500 nm. Thus the term "nanoparticle" encompasses both ultrafine particles (1 to 100 nm) and fine particles from 100 to 500 nm.

The term "linker molecule" as used herein, is taken to mean a molecule with at least one available functional group that can be coupled to the antimicrobial agent and /or a nanopaticle surface. In embodiments of the invention, the linker molecule has at least one available functional group that can be coupled to the antimicrobial agent and at least one available functional group that can be coupled to the nanoparticle surface and thus is retained in the resulting antimicrobial-nanoparticle conjugate. In alternative embodiments of the invention, the 'linker molecule' may be a substance which activates a chemical moiety on the antimicrobial agent or the nanoparticle to enable bond formation therebetween. The linker molecule may itself be an activated functionality, such as a terminal amine linkage on a nanoparticle, or the primary amine of an antimicrobial agent, which can form a direct linkage between the nanoparticle and the antimicrobial agent.

In an embodiment of the invention, the antimicrobial agent is covalently immobilized to the nanoparticle by the linker molecule. The linker molecule may be an activated polymer. Activated polymers are useful linker molecules due to the number of sites available for activation.

In an embodiment of the invention, the concentration of the antimicrobial agent present on the surface of the nanoparticle is a function of the degree of activation of the polymer. Thus, the degree of functionalization of the polymer can be adjusted in order to control the amount of antimicrobial agent immobilized thereto.

In an embodiment of the invention the polymer is a polysaccharide. Polysaccharides are natural products and are usually biocompatible, making them particularly useful for use in biomedical applications. Any polysaccharide suitable for acting as a linker molecule between the antimicrobial agent and the nanoparticle can be employed. Examples of suitable polysaccharides include dextran, hyaluronic acid, arabinogalactan, xanthan, agarose, alginate, inulin, cellulose, starch, chitosan and chitin.

In an embodiment of the invention, the polysaccharide is dextran. Dextran is a branched glucan which is available in a wide range of molecular weights. Dextran contains a high density of hydroxyl groups which are capable of further chemical functionalization, allowing the degree of activation and thereby the concentration of the antimicrobial agent immobilized to be readily controlled. In addition, the presence of the hydroxyl groups renders dextran highly hydrophilic, which is advantageous when used as a linker molecule for hydrophobic antimicrobial agents, as it can increase the solubility of the antimicrobial conjugate in aqueous solution, thereby facilitating its immobilization into the nanoparticles. As a further advantage, studies have demonstrated that surface coatings comprising dextran may be capable of limiting protein and cell adhesion to surfaces (Frazier et al. Biomaterials 2000, 21, 957-966).

Suitable nanoparticles for incorporation in the antimicrobial nanoparticle conjugates of the invention include, but are not limited to, gold nanoparticles, silver nanoparticles, silica nanoparticles and organic nanoparticles such as poly(lactic acid-co-glycolic acid), polyethyleneimine, chytosan and poly(hyaluronic acid).

In an embodiment of the invention, the nanoparticle is a silica nanoparticle. Silica nanoparticles are particularly advantageous for the formation of stable antimicrobial conjugates due to the absence of cytotoxic response in patients, high stability, durability, and efficient functionalization.

Nanoparticles for immobilization in the conjugate can be of any suitable diameter. In particular, the average diameter of the nanoparticles can be from 2 to 500 nm; from 10 to 400 nm; from 20 to 350 nm. In an embodiment, the average diameter of the nanoparticle can be from 20 to 100 nm.

In an embodiment of the invention, the antimicrobial conjugate comprises at least one functional amine group. For example, antimicrobial agents comprising an amino sugar such as amphotericin B and nystatin are particularly suitable as the amino group of the sugar can form imine bonds with suitable functional groups of the linker molecule.

The antimicrobial agent can be an antibiotic or antifungal. In an embodiment, the antifungal agent can be a polyene antifungal or polycationic compound. Suitable polyene antifungals include, but are not limited to, amphotericin B, natamycin, rimocidin, nystatin, filipin, candicin and hamycin. Suitable polycationic compounds may have quaternary amines and include, for example, alkylated chitosan and alkylated polyethyleneimine.

In an embodiment of the invention, the polyene antifungal is amphotericin B.

In an aspect of the invention there is provided a process of preparing an antimicrobial nanoparticle conjugate as described above comprising the steps of:
- providing an activated-linker-antimicrobial conjugate; and
- coupling the conjugate to a nanoparticle.

Use of an activated linker-antimicrobial conjugate in the preparation of antimicrobial-nanoparticle conjugate is envisaged. In an embodiment, an oxidised dextran-amphotericin B conjugate can be used in the preparation of an amphotericin B-nanoparticle conjugate. In this embodiment, the nanoparticle can be a silica nanoparticle.

In an aspect of the invention there is provided a method of immobilizing an antimicrobial agent to a nanoparticle comprising the steps of:
functionalizing the nanoparticle;
preparing an activated linker;
reacting the activated linker with the antimicrobial agent to form an activated-linker-antimicrobial conjugate;
   and
reacting the activated-linker-antimicrobial conjugate with the functionalized nanoparticle to form an antimicrobial-functionalized nanoparticle.

The nanoparticle may be functionalized by any means suitable for providing the nanoparticle with an available functional group for conjugation to the activated linker. In an embodiment, the nanoparticle is functionalized via silanization to provide terminal amine groups at the nanoparticle surface, which are capable of binding with the activated linker.

The silanization may be carried out by any known method. In an embodiment, the silanization is performed with a mixture of silanizing agents. In an alternative embodiment, the silanization is performed with a single silanization agent.

The activated linker may be prepared by any known method to functionalize a suitable molecule such that conjugation between the antimicrobial agent and the nanoparticle can occur. In an embodiment, the activated linker is prepared by oxidation of the linker molecule. In an embodiment, oxidation of the linker molecule yields functional aldehyde groups.

In an embodiment, terminal amine groups of a silanized nanoparticle can conjugate to the activated linker by the formation of imine bonds with available aldehyde groups on the activated linker.

In an embodiment, the method further comprises the step of:
reducing the antimicrobial-functionalized nanoparticle to form a stable antimicrobial nanoparticle conjugate.

The reducing step may be performed by any suitable reducing agent, such as, for example, sodium cyanoborohydride.

In an embodiment, at least one of the steps of reacting the activated linker with the antimicrobial agent and reacting the activated-linker-antimicrobial conjugate with the functionalized nanoparticle is carried out in aqueous solution. In another embodiment, both of these steps are carried out in aqueous solution. Any suitable aqueous solution can be used, for example, borate buffer. Advantageously, the use of organic solvents, which may be harmful to the environment, can be avoided.

In an embodiment of the invention, the activated linker is an oxidized polysaccharide. Oxidized polysaccharides are particularly suitable for conjugation in the methods of the invention due to the large number of aldehyde groups on the backbone of the polysaccharide which are available for conjugation with the antimicrobial agent and the nanoparticle.

In an embodiment of the invention, the degree of oxidation of the polysaccharide is manipulated in order to control the amount of antimicrobial agent immobilised thereto.

The "degree of oxidation (DO)" as used herein, is defined as the number of oxidized residues per 100 glucose residues and this value can be determined, for example, by using *t*-butyl carbazate (tBC) as described in Boudahir *et al* (Boudahir et al. Polymer 1999, 40, 3575-3584). Briefly, in this technique, the *t*-butyl carbazates are reacted with aldehyde groups to form carbazones. After reacting an excess amount of *t*-butyl carbazate with the oxidised polysaccharide, the unreacted carbazates can be quantified by adding trinitrobenzenesulfonic acid (TNBS) and measuring spectrophotometrically the resulting trinitrophenyl derivative at 334 nm. By subtracting the number of unreacted carbazates from the total number of added carbazates, the number of aldehydes in the solution can be determined.

In an embodiment, the degree of oxidation is from 1 to 70%. Above this degree of oxidation, the polysaccharide may be cleaved by the oxidising agent during the oxidation reaction, therefore presenting as low molecular weight polymers which are less able to immobilize high concentrations of the antimicrobial agent per polymer molecule.

In an embodiment of the invention the degree of oxidation is from 1 to 50%. In an embodiment of the invention the degree of oxidation is from 1 to 30%.

Oxidizing the polysaccharide to a DO of up to 70% advantageously allows immobilization of a significant amount of the antimicrobial agent into the backbone of the polymer, while the remaining aldehyde groups can be used for the attachment of the polysaccharide-antimicrobial conjugate onto the surface of the nanoparticle.

In a preferred embodiment of the invention, the DO of the polysaccharide is from 20 to 30%.

Preferably the oxidized polysaccharide is oxidized dextran.

Dextran can be oxidised by any known process such as that described in Maia et al., Polymer 2005, 46(23) 9604-9614 which uses sodium periodate. In this process, the periodate ion attacks one of the hydroxyl groups of the vicinal diol in dextran residues, between C₃-C₄ or C₂-C₃, breaking the C-C bond and yielding two aldehyde groups.

In an embodiment of the invention, the antimicrobial agent is amphotericin B. When amphotericin B is used as the antimicrobial agent with oxidised dextran as the linker molecule, the step of reacting the oxidised dextran with amphotericin B results in imine bond formation between the primary amine of the amphotericin B sugar moiety and an available aldehyde group of the oxidized dextran.

The reaction can be controlled to ensure that not all of the available aldehyde groups on the oxidised dextran are conjugated to amphotericin B, so that sufficient aldehyde groups on the oxidised dextran remain available for conjugation to the nanoparticle.

After conjugation to the nanoparticle, the imine bonds may then be reduced to form stable secondary amines.

In an aspect of the invention there is provided use of an antimicrobial nanoparticle conjugate as described above as an antimicrobial coating for a medical, dental or surgical device or material. The method may comprise coating at least one surface or a part of a surface of the device or material with an antimicrobial nanoparticle conjugate as described herein.

The conjugate may be utilised in the form of a suspension of the nanoparticle conjugate in any suitable solution.

According to an aspect of the invention there is provided a method of preparing a medical, dental or surgical device or material with an antimicrobial coating, wherein the method comprises coating at least one surface or part of a surface of the device or material with an antimicrobial nanoparticle conjugate described herein.

The at least one surface or part of a surface of the device or material can be coated by any suitable means known in the art such as, for example, spray-drying, brushing or dipping.

In an embodiment of the invention, the at least one surface or part of a surface is treated with an adhesive prior to coating. Treating the surface with an adhesive prior to coating can ensure efficient immobilization of the antimicrobial nanoparticle conjugate. The adhesive may be a chemical adhesive.

In an embodiment, the adhesive is formed by a polyelectrolyte of opposite charge to the antimicrobial nanoparticle conjugate which is adsorbed or chemically reacted with the substrate. In this case, assembly of the nanoparticles into large-area uniform thin films can be achieved by layer-by-layer techniques such as described in Lee D et al., Langmuir 2007, 23, 8833.

In an alternative embodiment, the adhesive is formed by nanoparticles of opposite charge to the antimicrobial nanoparticle conjugate. These nanoparticles can interact with the substrate by electrostatic interactions and physically immobilize the antimicrobial nanoparticle conjugates of opposite charge.

In an embodiment, the adhesive is a chemical adhesive formed by polydopamine. Dopamine, a biomolecule that contains catechol and amine functional groups, found also in high concentration in mussel adhesive proteins, polymerizes at alkaline pHs to form thin adherent polydopamine films that exhibit latent reactivity toward amine and thiol groups (Lee H et al, Advanced Materials 2009, 21, 431).

The method of the invention can be used to apply an antimicrobial coating to any suitable surface such as, for example, metals ceramics, polymers, fibres and glass. Suitable metals include, for example, titanium and titanium alloys such as nitinol, nickel-titanium alloys and thermo-memory alloy materials; stainless steel; tantalum; nickel-chrome alloys and cobalt alloys such as cobalt-chromium alloys Elgiloy® and Phynox®. Suitable ceramic materials include, for example, oxides, carbides or nitrides of the transition elements such as titanium oxides, hafnium oxides, iridium oxides, chromium oxides, aluminium oxides and zirconium oxides. Suitable polymers include styrene and substituted styrenes, ethylene, propylene, poly(urethanes), acrylates, methacrylates, acrylamides, methacrylamides, polyesters, polysiloxanes, polyethers, poly(orthoester), poly(carbonates), poly(hydroxylalkanoates), and copolymers thereof.

Surfaces which may be coated can be in the form of films, particles, beads and fibres.

In embodiments of the invention, the surface may be an absorbent or fibrous material, such as those used for cotton wools, gauzes, bandages, swabs, lints, tapes, sanitary napkins and towels, surgical dressing, sponges, compresses, plasters and surgical garments such as aprons, masks, caps and gowns. Advantageously, when the antimicrobial conjugate is applied as a coating to such materials, which may be disposable, the coating will maintain its antimicrobial activity even after disposal, which can help minimize contamination from waste materials.

In an aspect of the invention there is provided a medical, surgical or dental device or material with an antimicrobial coating, obtainable by the method as described herein.

In an aspect of the invention there is provided a medical, surgical or dental device or material comprising at least one surface or part of a surface coated with, or formed from a material containing, an antimicrobial nanoparticle conjugate as described herein.

In an embodiment, the device may be an implantable or injectable device. In an embodiment, the implantable or injectable device may be selected from the group consisting of intra-ocular lenses, stents, catheters, scaffolds, tubing, needles, pacemakers, prosthetics, bone cements, screws, rivets, plates, valves, grafts, contraceptive devices, sensors and pumps.

The device or material may be selected from the group consisting of hand-held surgical and dental instruments, meters, monitors, gauze, dressings, sanitary pads and towels, wound dressings, surgical drapes, masks and garments, diapers and sponges.

In an embodiment, the antimicrobial agent may be an antifungal. In this embodiment, the surface or part of a surface may have an antifungal activity at or below an immobilized concentration of 10µg/cm² of the antifungal. The surface or part of a surface may have an antifungal activity at or below an immobilized concentration of 5 µg/cm² of the antifungal. The surface or part of a surface may have an antifungal activity at or below an immobilized concentration of 3 µg/cm² of the antifungal.

In an embodiment of the invention, the antimicrobial nanoparticle conjugate is substantially non-hemolytic. The term "substantially non-hemolytic" as used herein means that the substance or conjugate does not induce a significant amount of hemolysis of red blood cells. In an embodiment, the substance or conjugate does not induce more than 50% of hemolysis of red blood cells.

In an embodiment of the invention, the antimicrobial nanoparticle conjugate is substantially non-cytotoxic. The term "substantially non-cytotoxic" as used herein means that the substance or conjugate does not reduce mononuclear cell metabolism more than 20% after 24 hours of exposure to 1 cm² of surface containing or coated with the antimicrobial nanoparticle conjugate.

In addition to the antimicrobial agent, it is envisaged that one or more pharmaceutically active agents in the form of proteins and small organic or inorganic molecules could also be coupled to the conjugate, or directly to the surface of the medical device or material. Such agents may include, for example, antiproliferative, cytostatic or cytotoxic chemotherapeutic agents, antiinflammatories, growth factors, cell adhesion peptides and agents which inhibit scarring. These agents may be attached to the conjugate or directly to the surface using a hydrolysable linkage, to promote controlled release of the active agent, for example at the site of insertion or implantation of a medical device.

It is also envisaged that the antimicrobial conjugates of the invention could be of use outside of the medical field, particular for example, in the context of paints and coatings for which antimicrobial, and especially antifungal activity, is desirable.

The invention is now further described with reference to the following figures:
Figure 1: Scanning electron microscopy and transmission electron microscopy images of silica nanoparticles prior to conjugation or suspension of the nanoparticles.
Figure 2: UV-VIS spectra of amphotericin B, Oxidised dextran and Oxidised dextran-amphotericin B conjugate, in accordance with the present invention.
Figure 3: ¹H-NMR spectra of amphotericin B, Oxidised dextran and Oxidised dextran-amphotericin B conjugate, in accordance with the present invention.
Figure 4: Size distribution of amphotericin B -oxidised dextran-silica nanoparticle conjugates in accordance with the present invention.
Figure 5: Yeast killing by amphotericin B -oxidised dextran-silica nanoparticle conjugates, in accordance with the invention.
Figure 6: Contact-mediation of antifungal activity of amphotericin B-oxidised dextran-silica nanoparticle conjugates, in accordance with the invention.
Figure 7: Yeast growth kinetics in the presence of nanoparticles.
Figure 8: Stability of nanoparticle suspensions over time.
Figure 9: Yeast killing by silver nanoparticles.
Figure 10: Yeast killing by amphotericin B -oxidised dextran-silica nanoparticle (5 nm) conjugate immobilized on a surface, in accordance with the invention.
Figure 11: Percentage of lysed blood cells after contact with coverslips coated with amphotericin B-oxidised dextran-silica nanoparticle conjugates, in accordance with the invention.
Figure 12: Mitochondrial metabolic activity of mononuclear cells plated on top of coverslips with different coatings. Results are average ± SD, n=6. Each absorbance at 540 nm was normalized by the one obtained on glass coverslip.

### Detailed description of the invention:

The invention is directed to antimicrobial nanoparticle conjugates comprising an antimicrobial agent immobilized to a nanoparticle by a linker molecule. By way of illustration only, the invention will now be fully described in the context of an amphotericin B(AmB)-silica nanoparticle (SNP) conjugate immobilized via oxidized dextran. A schematic illustration of the preparation of the nanoparticle conjugates is illustrated below:

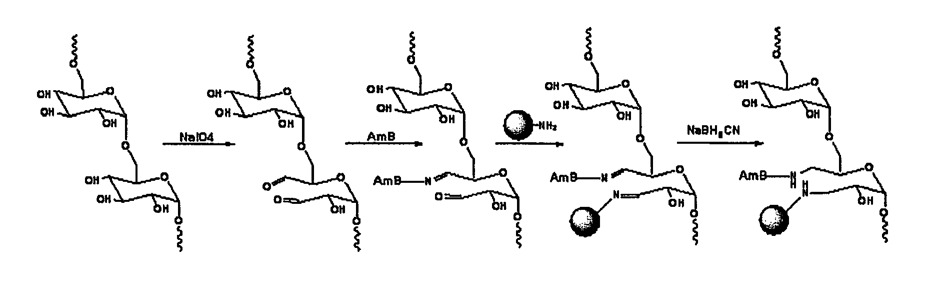

In the above method dextran is firstly oxidised with sodium periodate, breaking the C-C bond of the glucopyranoside and yielding two aldehyde groups. The oxidised dextran (DexOx) is then reacted with AmB in aqueous solution to yield an oxidised dextran-amphotericin B conjugate (DexOx-AmB). The DexOx-AmB conjugate is reacted in aqueous solution with silanized silica nanoparticles comprising terminal amine groups (SNP-NH₂), such that coupling of the DexOx-AmB conjugate to the silica nanoparticle can occur via imine bond formation between the primary amine of the AmB and available aldehyde groups of the oxidised-dextran. Reduction of the unstable imine bonds yields a stable secondary amine.

Although the above is written in the context of the conjugation of a DexOx-AmB conjugate to a nanoparticle, it will be appreciated that the activated linker could be conjugated firstly to a nanoparticle to create an activated-linker-nanoparticle conjugate, and thereafter to an antimicrobial agent, i.e. a DexOx-SNP-NH2 conjugate could be prepared and then immersed in a solution of AmB to create an AmB-nanoparticle conjugate.

The invention will now be described by way of illustration only in the following examples:

### Example 1: Preparation and characterisation of silica nanoparticles

Silica nanoparticles with diameters of 5 nm, 80 nm and 170 nm (SNP5, SNP80, SNP170) were used. Commercially available silica nanoparticles of 5 nm and 80 nm in diameter were purchased from Eka Chemicals AB (Bohus, Sweden) and PlasmaChem GmbH (Berlin, Germany), respectively, while nanoparticles of 170 nm diameter were prepared as detailed below.

### 1.1 Preparation of silica nanoparticles of 170 nm diameter

Fluorescent-labelled nanoparticles of 170 nm in diameter were prepared as described in Nyffenegger et al., Journal of Colloid and Interface Science 1993, 159 (1), 150-157. Briefly, in a reaction vessel protected from light, fluorescein isothiocyanate (FITC) (39 mg, 9.8 x 10⁻⁵ mol, Sigma) and (3-aminopropyl)triethoxysilane (APTES) (Sigma) (20 µl, 1.13 x 10⁻⁴ mol) were mixed in methanol (100 ml, 79g) and reacted for two days, at 42°C, under stirring, yielding APTES conjugated with FITC (APTES-FITC). The nanoparticle seeds were formed by adding 330 µl of tetramethyl orthosilicate (TMOS) (Sigma) in 49 ml of ammonia-methanol solution (127 ml of methanol in 21.5 ml of ammonia 11.6M), under magnetic agitation, for 50 minutes, at 25°C. Then, 20g of the seed suspension (25 ml) was diluted with 25 ml of the ammonia-methanol solution and 2g of APTES-FITC solution was added. A total amount of 330 µl of TMOS was added in 20 portions of 16.5 µl. The time between each addition was about 8 minutes, in order to allow sufficient time for completion of the reaction.

### 1.2 Characterisation of Nanoparticles

Silica nanoparticles having initial diameters of 5 nm, 80 nm and 170 nm were characterised prior to conjugation. Accordingly, the term "initial diameter" refers to the diameter of the nanoparticle prior to conjugation or suspension. Nanoparticles were characterised by scanning electron microscopy and transmission electron microscopy as detailed below.

### 1.2.1 Scanning electron microscopy (SEM) analysis

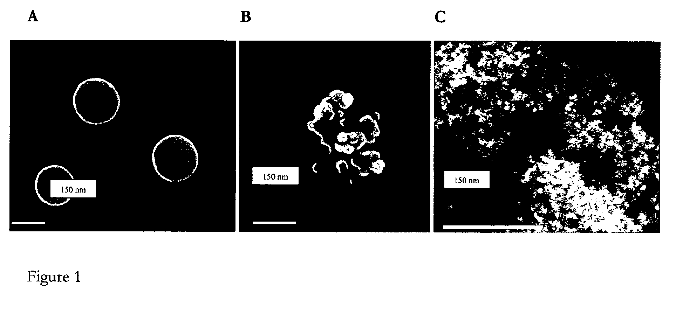

Nanoparticles suspended in 2-(N-morpholino)ethanesulfonic acid (MES) buffer (0.008 mg/ml) were deposited on 0.5 cm² glass slides. The solvent was allowed to evaporate and the slides mounted on a SEM sample stub using conductive carbon cement. The samples were then carbon coated by plasma vapour deposition and analyzed by a Hitachi S4100 SEM. Results of the SEM analysis are shown in Figures 1 (A) and 1(B).

### 1.2.2 Transmission electron microscopy (TEM) analysis

For TEM analysis, the suspension of NPs (8 µg/ml, in hexane) was spray coated on a TEM 400 mesh grid. The NPs were then observed by TEM on a JEOL JEM-100 SX microscope at 100 kV. Results of the TEM analysis are shown in Figure 1 (C).

SEM and TEM images of the nanoparticles illustrated in Figure 1 show the nanoparticles have a relatively uniform diameter.

Silica nanoparticles of initial diameter 5 nm, 80 nm (SNP5, SNP80) were then suspended in an aqueous solution of distilled water to determine the effect of the suspension on particle diameter. In suspension in aqueous solution, the SNP5, SNP80 and SNP170 had diameters of 1 nm, 79 nm and 174 nm, respectively, as illustrated in Table 1.

**Table 1: Effect of suspension in aqueous solution on nanoparticle diameter**

| **Silica Nanoparticles** | **Diameter(nm) /polydispersity *** | **Zeta potential (mV)**** |
|---|---|---|
| SNP5 | 1.2 ± 0.2/(0.323) | -12.9 ± 1 |
| SNP80 | 78.5 ± 2.5/(0.337) | -11.2 ± 2.8 |
| SNP170 | 174.3 ± 8.7/(0.062) | -22.7 ± 2.9 |

| | | |
|---|---|---|
| * Average mode of three measurements. All measurements were performed in distilled water. The diameter is measured in nm and the polydispersity is calculated as a % value. ** Zeta potential of the particles was measured in a diluted suspension (0.5 mg/ml) in 0.1 M MES buffer pH 5.5. The results are the average of three measurements. | | |

### Example 2: Silanization of silica nanoparticles and characterisation of silanized nanoparticles

### 2.1 Effect of silanization agent

In order to determine the effect of different silanization agents, the effect of freeze-drying and re-suspension on the diameter of silica particles which had been functionalized with different silanization agents was determined. Silanization was performed with (trihydroxysilyl)propylmethylphosphonate (THPMP), a 1:1 mixture of (3-aminopropyl)trimethoxysilane (APTMS) and 3-(trihydroxysilyl)propylmethylphosphonate (THPMP); and a 1:4 mixture of (3-aminopropyl)trimethoxysilane (APTMS) and 3-(trihydroxysilyl)propylmethylphosphonate (THPMP). For comparison, unsilanized nanoparticles were also included in the study, the results of which are shown in Table 2 below:

**Table 2: Effect of silanization agent on nanoparticle diameter**

| **Silanization** | **Initial diameter (nm)/(polydispersity)** | **Diameter after silanization and freeze drying (nm)/ polydispersity*** |
|---|---|---|
| No treatment | 155 (0.155) | 1108 (0.124) |
| THPMP | 138 (0.189) | 188 (0.165) |
| APTMS:THPMP (1:1) | 150 (0.248) | 622 (0.257) |
| APTMS:THPMP (1:4) | 134 (0.267) | 214 (0.171) |

| | | |
|---|---|---|
| * Sonication for 40 minutes followed by vortex. | | |

From this data it is evident that the use of 1:4 (v/v) AMPTS:THPMP as silanization mixture advantageously stabilizes the nanoparticle suspension and prevents aggregation of the nanoparticles. A 1:4 (v/v) mixture of AMPTS:THPMP was therefore chosen for silanization of the SNP80 and SNP170 nanoparticles in further methods of the invention.

### 2.2. Silanization of silica nanoparticles:

### SNP170

Silanization of the SNP170 nanoparticles prepared above was performed using a 1:4 (v/v) mixture of AMPTS:THPMP as described in example 2. Silanization was performed directly in methanol/ammonia solution (25%, 6:1 v/v) with 2.5% (v/v) of APTMS and THPMP (1:4) under magnetic stirring at room temperature for 3 hours. The nanoparticles were then centrifuged at 3,200 g for 10 minutes and washed three times with 10 mM 2-(*N*-morpholino)ethanesulfonic acid (MES) buffer pH of 5.5.

### SNP80

Silanization of the SNP80 nanoparticles was performed as for the SNP170, with the exception that the nanoparticles were centrifuged at 20,000 g for 40 minutes.

### SNP5

Silanization of the 5 nm silica nanoparticles was performed using a single silanization agent. Briefly, a commercially-available SNP5 suspension was diluted with borate/NaOH buffer pH 10.8 to yield a final concentration of 7.5 mg/ml, refluxed under vigorous magnetic stirring (750 rpm) in the presence of 3-aminopropyldimethylinethoxysilane (APMMS) (87 µl in 1 ml borate buffer, corresponding to 5% of the weight of SNP5 in the suspension) for 3 hours.

### 2.3 Characterization of silanized nanoparticles:

The silanized silica nanoparticles were then characterised in terms of amino groups, net charge and particle diameter, as described in more detail below. Results are illustrated in Table 3.

**Table 3: Characterisation of silanized silica nanoparticles**

| **Silanized Silica Nanoparticles** | **Diameter (nm)/polydispersity*** | **[NH₂](mol/mg nanoparticles)**** | **Zeta potential (mV)***** |
|---|---|---|---|
| SNP5-NH₂ | 12.0 ± 2.0/(0.289) | 2.2 x 10⁻⁸ | +15.0 ± 1.1 |
| SNP80-NH₂ | 215.4 ± 23.1/(0.158) | 4.0 x 10⁻⁸ | +28.8 ± 0.6 |
| SNP170-NH₂ | 185.2 ± 23.0/(0.232) | 1.7 x 10⁻⁹ | +10.9 ±0.7 |

| | | | |
|---|---|---|---|
| * Average mode of three measurements. All measurements were performed in distilled water. The diameter is measured in nm and the polydispersity is calculated as a % value. ** Zeta potential of the particles was measured in a diluted suspension (0.5 mg/ml) in 0.1 M MES buffer pH 5.5. The results are the average of three measurements. | | | |

### 2.3.1 Quantification of amino groups

The concentration of amino groups on the surface of the silica nanoparticles was quantified by a ninhidrin assay as described in Moore, S et al. J Biol Chem., 1954, 211(2), 907-913. Briefly, in a scintillation vial, 1 ml of nanoparticle suspension (20 mg/ml in water) was added to 1 ml of ninhydrin reagent, mixed and then immersed in boiling water. After 15 minutes, the vials were removed from the water bath and 15 ml of an ethanol/water mixture (1:1, v/v) was added to the reaction, which was then allowed to cool at room temperature for 15 minutes, in the absence of light. The particle samples were centrifuged before reading to avoid the interference of suspension turbidity in the absorbance reading. The absorbance at 570 nm was converted into concentration by using a calibration curve with solutions of glycine (10-45 mM).

As expected, the concentration of amine groups per mg of nanoparticles was shown to increase significantly for smaller nanoparticles. Consequently, the net charge for SNP5 and SNP 80 was positive, but not for SNP170.

### 2.3.2 Calculation of net charge

The zeta potential of the nanoparticles was determined by a Zeta Plus analyzer. A 20 µl aliquot of nanoparticles suspended in ethanol (8mg/ml) was added to 1.5 ml of 0.1 M MES buffer pH 5.5 and the zeta potential determined. Average values were based on three independent measurements.

### 2.3.3 Diameter of nanoparticles

The average diameter of the silanized nanoparticles (SNP5-NH₂, SNP80-NH₂, SNP170-NH₂) suspended in 2ml filtered milliQ® water dispensed through a 0.22 µm membrane filter, was determined by dynamic light scattering method (DLS) using a Zeta Plus analyser. Average values were based on six independent measurements.

### Example 3: Preparation of oxidized dextran (DexOx)

Oxidized dextran was prepared according to the method described by Maia et al., Polymer 2005, 46(23) 9604-9614 using sodium periodate. Briefly, dextran (Mw ~ 70,000 Da) was oxidized in aqueous solution with an amount of sodium periodate calculated to yield 25% oxidation, for 20 hours at room temperature. The product was then dialysed for 48 hours using a dialysis membrane with a molecular weight cut-off of 6-8,000 Da, against Millipore® water (dispensed through a 0.22 µm membrane filter), at 4°C, in the dark. Yields above 90% were obtained. The degree of oxidation was determined colorimetrically by a TNBS assay and ¹HNMR as described in Maia et al., Polymer 2005, 46(23) 9604-9614. The degree of oxidation was determined to be 23%. A degree of oxidation of 23% allows the immobilization of a significant amount of amphotericin B into the backbone of the polymer (<15%), while the remaining aldehyde groups can be used for the attachment of the conjugate into the surface of the nanoparticle containing terminal amine groups.

### Example 4: Preparation and characterisation of a polysaccharide-antifungal conjugate of amphotericin B with oxidised dextran (AmB-DexOx)

### 4.1 Preparation of AmBDexOx

A solution of oxidised dextran (10 - 30 mg/ml, dissolved in 0.1 M borate buffer pH 10.2) was mixed with a solution of AmB (in DMSO or 0.1 M borate buffer pH 10.8 to improve the solubilization of the AmB during the reaction) with a predefined concentration, in order to obtain a theoretical degree of derivatisation of 4, 17 and 30 %. The reaction was allowed to proceed for 18 hours at room temperature, under magnetic stirring, and shielded from light. Then, a sodium dodecyl sulphate (SDS) aqueous solution (10 ml, 10 mM) was added to the reaction vial in order to prevent the aggregation of unreacted AmB and the solution transferred to a dialysis membrane with a molecular weight cut-off of 6-8,000 Da. The dialysis was performed in the dark, at 4°C, for 48 hours against 10 mM SDS aqueous solution as described in Stoodley et al., Langmuir 2007, 23(17), 8718-8125. The aqueous SDS solution was changed twice daily. The total volume of dialysate was then freeze-dried and weighed. The reaction yield was approximately 81%.

### 4.2 Characterisation of AmBDexOx

### 4.2.1 UV-VIS

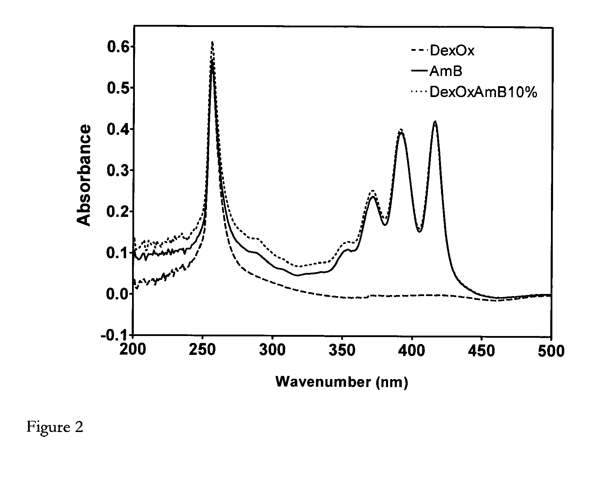

Coupling of AmB to DexOx was initially confirmed by UV-VIS absorption (Figure 2). Peaks at 416, 391 and 371 nm confirmed the presence of AmB. The amount of AmB immobilized into the DexOx was determined spectrophotometrically by the quantification of AmB peak intensity at 416 nm. The conjugate was solubilised in DMSO and the calculations were performed based on a calibration curve of AmB in DMSO (0-16 µg/ml).

### 4.2.2 ¹H-NMR

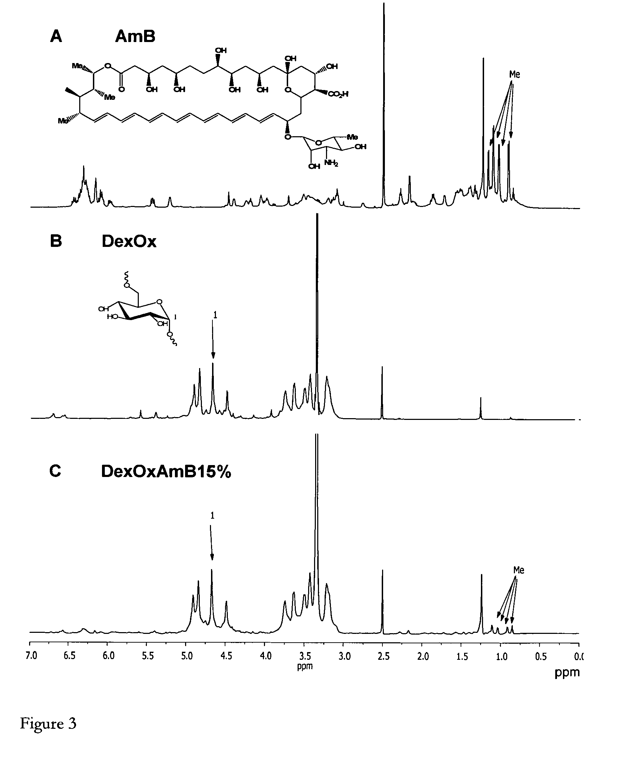

The coupling of AmB to DexOx was further confirmed by ¹H-NMR. The NMR spectrum shows peaks between 0.75 and 1.2 ppm corresponding to the methyl groups of AmB, and peaks between 3.0 and 5.0 corresponding to the protons of glucose residues of dextran (Figure 3). The ratio of AmB to DexOx was obtained by calculating the ratio between 1 /3 of the peak at 1.2 ppm (protons of a methyl group of AmB) and the area of the peak corresponding to the anomeric proton of dextran, located at 4.67 ppm. The area of the peak was integrated using Nuts Pro Software (Acorn NMR Inc.).

Three DexOx-AmB conjugates were prepared by the above method having 5, 10 and 15% respectively of AmB per 100 dextran glucopyranoside residues (hereinafter denoted DexOx-AmB5, DexOc-AmB10 and DexOx-AmB-15). All of the conjugates presented a higher solubility in water (at least 12 mg/ml, maximum concentration tested; corresponding to a concentration of 3.5 mg/ml of AmB) than unconjugated AmB for which a water solubility limit of 0.1 mg/ml has been reported (Budvari et al., The Merck Index: an encyclopaedia of chemicals, drugs and biologicals. Merck & Co. New York, 1989).

### 4.3 Determination of Antifungal activity of DexOx-AmB conjugates:

To evaluate the antifungal activity of the DexOx-AmB conjugates, the minimal inhibitory concentration (MIC) was determined for the DexOx-AmB conjugates with variable AmB incorporation (5%, 10% and 15%) and compared with values obtained for AmB. The "minimal inhibitory concentration" or "MIC" is defined as the lowest drug concentration that results in complete inhibition of visible growth of a microorganism. The tests were performed against *Candida albicans* (n=6), at 30°C, for 48 hours, according to the National Committee for Clinical Laboratory Standard (NCCLS) [M27A2E] guidelines. AmB had a MIC of 0.9 µg/ml for *Candida albicans,* similar to that reported in the literature (Canton et al., Antimicrob Agents Chemother 2004, 48(7) 2477-2482.

As can be seen from Table 4 below, DexOx-AmB conjugate with a degree of AmB incorporation of 15% had a similar MIC to the unconjugated AmB (0.9 ± 0.2 versus 1.2 ± 0.6 µg of immobilized AmB per ml of media; in both cases n=6).

**Table 4: Minimal inhibitory concentration (MIC) for AmB and DexOx-AmB conjugates with variable AmB incorporation**

| **Degree of AmB incorporation** | **MIC (µg of conjugate/ml)** | **MIC (µg of immobilized AmB/ml)** |
|---|---|---|
| AmB | 0.9 ± 0.2 | not applicable |
| DexOx23%-AmB5% | 6.5 ± 2.8 | 1.9 ± 1.0 |
| DexOx23%-AmB10% | 0.8 ± 0.2 | 0.5 ± 0.1 |
| DexOx23%-AmB15% | 1.5 ± 0.7 | 1.2 ± 0.6 |

### Example 5: Immobilization of DexOx-AmB to SNP and characterisation of SNP-DexOx-AmB conjugates

### 5.1 Immobilization of DexOx-AmB to SNP

Silica nanoparticles (20 mg) silanized with AMPES and THPMP (1:4, v/v) as described above, were re-suspended in 1ml 0.01 M borate buffer pH 10.2. As a control, silica nanoparticles which had not been silanized were also used. The coupling reaction of DexOx-AmB with silica nanoparticles was initiated by adding 3 ml of DexOx-AmB solution (4 mg in 0.01 M borate buffer pH10.2) under agitation and allowed to proceed for approximately 18 hours. Then, sodium cyanoborohydride (5 x 10⁻⁴ mol, 10 x excess to the imine bonds) was added for 1 hour to reduce the imine bonds. For SNP170, the whole reaction volume was centrifuged for 5 minutes at 10,000 rpm (Avanti J-26 XPI) and the pellet re-suspended in 2ml milliQ ® water dispensed through a 0.22 µm membrane filter. The nanoparticles were then transferred to eppendorf tubes and washed three times with 2 ml of water using centrifugation steps between each wash. After this washing procedure the nanoparticles were immediately used for subsequent assays.

### 5.2 Quantification of immobilised DexOx-AmB

The content of DexOx-AmB immobilized onto the surface of the silica nanoparticles was determined by the anthrone colorimetric assay as described in Shields et al. Analytical Chemistry 1960, 32(7), 885-886. Briefly, this assay quantifies the amount of dextran immobilized into nanoparticles as follows: Nanoparticles conjugated to DexOx and suspended in water were centrifuged, washed with distilled water, centrifuged and finally re-suspended in water. The anthrone assay was performed on the supernatant after each washing step. For supernatants with negligible absorbance at 620 nm (i.e. < 0.05), the nanoparticle suspension was then tested with the anthrone assay. In this case, aliquots (4 ml) of anthrone solution (0.2% w/v in 96% sulphuric acid) were added dropwise to 2 ml of the sample, previously cooled to 4°C by immersion in an ice bath for 45 minutes. After mixing the contents, the tightly closed scintillation vials were immersed in a water bath at 90°C for 16 minutes. The reaction was stopped by immersing the vials in ice. The solutions were transferred to cuvettes and their absorbance at 620 nm measured after 30 minutes in a Power Wave multiwell plate reader equipped with KC Junior Software (Bio-Tek). All measurements were performed in triplicate.

From Table 5 below, it is evident that nanoparticles of small size such as SNP5 and SNP80 immobilize higher concentrations of DexOx-AmB conjugate than larger nanoparticles such as SNP170. Depending on the initial concentration of DexOx-AmB, SNP80 immobilizes 26 to 70 µg of the conjugate, while SNP170 immobilizes 1.7 to 5.4 µgl of the conjugate per mg of SNP. Significantly, high immobilization yields were obtained for conjugates with low percentage of AmB and thus with high percentage of aldehyde groups available for reaction with the terminal amine groups of the silica nanoparticles. Moreover, higher amounts of DexOx-AmB were immobilised on silica nanoparticles exposed initially to high concentrations of the DexOx-AmB conjugate.

**Table 5: Quantification of immobilized DexOx-AmB in SNPs**

| **Nanoparticles** | **DexOx-AmB conjugate** | **Concentration of immobilized DexOx-AmB conjugate (µg of conjugate per mg of SNP)** | | |
|---|---|---|---|---|
| | | **1.5 mg/ml** | **6 mg/ml** | **12 mg/ml** |
| | DexOx23%-AmB 5% | 1.7 | 3.3 | 5.4 |
| **SNP170** | DexOx23%-AmB 10% | - | - | - |
| | DexOx23%-AmB 15% | 2.0 | 3.2 | - |
| | DexOx23%-AmB 5% | 26.1 | 49.0 | 59.2 |
| **SNP80** | DexOx23%-AmB 10% | - | - | - |
| | DexOx23%-AmB 15% | 30.4 | 61.4 | 70.0 |
| | DexOx23%-AmB 5% | 19.7 | 36.8 | 81.9 |
| **SNP5** | DexOx23%-AmB 10% | - | - | - |
| | DexOx23%-AmB 15% | 7.9 | 32.3 | 110.3 |

| | | | | |
|---|---|---|---|---|
| * The DexOx-AmB conjugate (1.5, 6 or 12 mg/ml) was reacted with silanized nanoparticles (20 mg/ml) for 18 hours in 0.01 M borate buffer pH 10.8, at room temperature under magnetic stirring. Imine bonds were stabilized in the same solution, by adding 10-fold excess of sodium cyanoborohydride relative to the imine groups and stirring for 1 hour. The nanoparticles were washed with milliQ® water dispensed through a 0.22 µm membrane filter until no yellow conjugate was visible in the supernatant. The particles were freeze-dried and the amount of dextran immobilized per mg of nanoparticles was determined by the anthrone colorimetric assay. | | | | |

### 5.3 Size distribution

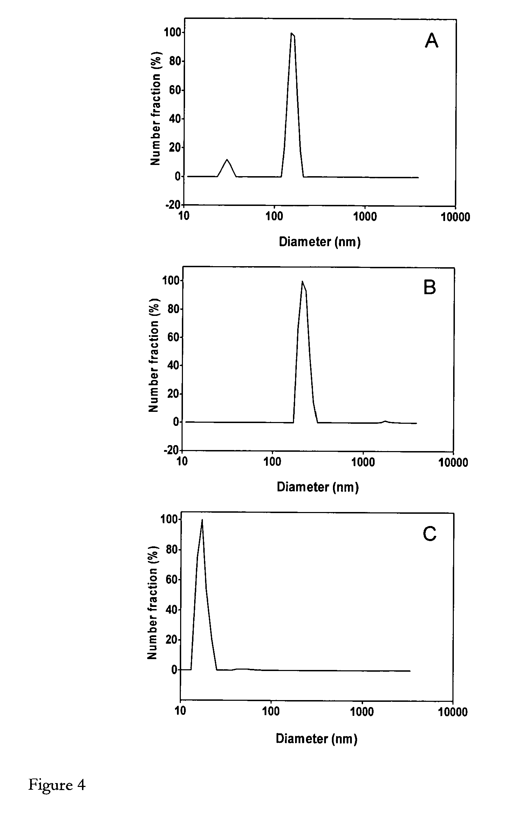

The size distribution by number of the silica nanoparticles functionalized with DexOx-AmB was determined by dispersing the conjugates (170 nm, 80 nm, 5 nm) in distilled water and sonicating before measurement. The results are shown in Figure 4, from which it can be seen that most of the SNP170 nanoparticles have diameters around 135 nm and a small population with diameters around 50 nm. SNP80 nanoparticles have a unimodal distribution around 330 nm, while SNP5 nanoparticles have a unimodal distribution around 22 nm.

### 5.4 Characterisation of SNP-DexOx-AmB Conjugates

The SNP-DexOx-AmB conjugates were then characterised in terms of diameter (nm) and zeta potential as described previously. Results are illustrated in Table 6.

**Table 6: Effect of conjugation on particle diameter**

| **Silica Nanoparticles***** | **Diameter (nm)/polydispersity*** | **Zeta potential (mV)**** |
|---|---|---|
| SNP5-NH₂-DexOxAmB | 22.0 ± 5.7/(0.349) | -12.5 ± 0.3 |
| SNP80-NH₂-DexOxAmB | 337.3 ± 60.6/(0.393) | -9.0 ± 1.6 |
| SNP170-NH₂-DexOxAmB | 134.8 ± 15.4/(0.233) | +10.9 ±0.7 |

| | | |
|---|---|---|
| * Average mode of three measurements. All measurements were performed in distilled water. The diameter is measured in nm and the polydispersity is calculated as a % value. ** Zeta potential of the particles was measured in a diluted suspension (0.5 mg/ml) in 0.1 M MES buffer pH 5.5. The results are the average of three measurements. *** The formulations were SNP-NH₂-(12mg/ml)DexOx23AmB15%. | | |

### Example 6: Assessment of the antifungal activity of the DexOx-AmB immobilized nanoparticles

### 6.1 Assessment of activity:

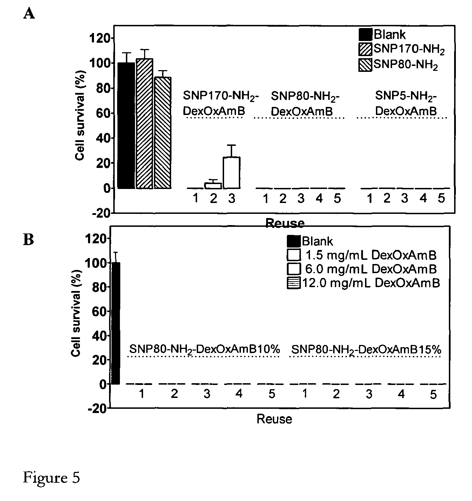

To assess the biological activity of the nanoparticles, 5 mg of nanoparticles functionalized with variable concentrations of the DexOx-AmB conjugates (DexOx-AmB 10% and 15%) were added to 1 ml of YPD media containing 1x10⁵ cells of *Candida albicans.* After 8 hours of exposure, an aliquot of the cell suspension was plated on YPD-agar plates for 14 hours, and the number of yeast colonies was counted. Figure 5 shows the cell survival % following the exposure. Figure 5A illustrates the cell survival percentage for silanized silica nanoparticles, i.e. those SNP5, SNP80 or SNP170 containing terminal amine groups (i.e. silanized nanoparticles) but without further modification to incorporate DexOx-AmB. It is evident from Figure 5a that these silanized nanoparticles have no significant antifungal activity.

In contrast, however, nanoparticles incorporating DexOx-AmB kill 100% of the microorganisms (Figure 5B). Nanoparticles SNP170 began to lose their antimicrobial activity after the first round of contact with *Candida.* This is likely due to the low concentration of AmB immobilized into the nanoparticles (i.e. 0.64 µg of AmB per mg of nanoparticles). However, five rounds of testing of the SNP5- or SNP80-DexOx-AmB conjugates showed no appreciable loss in activity demonstrating that significant reuse of the conjugates can be achieved.

The antifungal activity of the nanoparticles was unaffected by the initial concentration of DexOx-AmB used for the immobilization reaction. AmB incorporated in the nanoparticles was approximately 7.5 and 17 µg per mg of nanoparticles, when 1.5 mg/ml and 12 mg/ml of DexOx-AmB respectively were initially used, and thus above the MIC of Candida albicans (0.5 µg/ml, Table 7).

### 6.2 Mediation of Antifungal activity

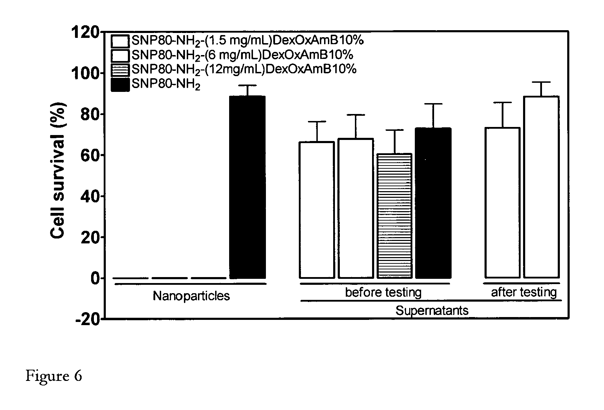

In order to assess the relative contributions from drug release and the nanoparticles themselves, the DexOx-AmB conjugated nanoparticles were incubated in YPD medium for 8 hours, centrifuged and the medium collected and tested against *Candida.* Figure 6 illustrates the cell survival % of *Candida* following the procedure.

From this figure it can be seen that limited yeast killing was observed (below 20%) for the incubation medium. Thus it can be deduced that the fungicidal activity of the nanoparticles is predominantly mediated by direct contact with the nanoparticle conjugate. This is significant for the use of these preparations as coating materials for medical devices, in which fungicidal activity based on contact with the coating is highly desirable.

### 6.3 MIC of SNP5 and SNP80-DexOx-AmB conjugates

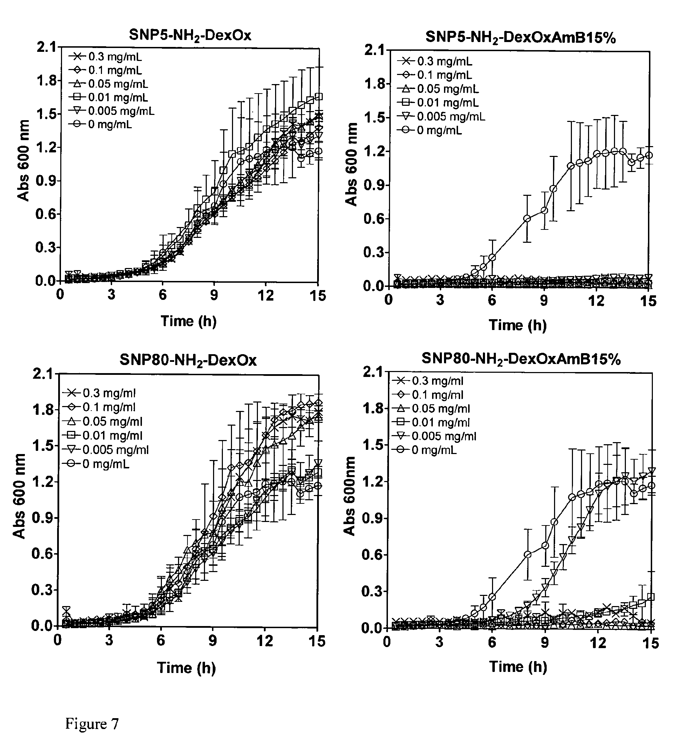

SNP5 and SNP80 were the most active nanoparticle conjugates and therefore the MIC of both conjugates was assessed. The MIC for SNP5 and SNP80 was 100 µg/ml and 300 µg/ml, respectively. At these concentrations, SNP5-DexOx-AmB and SNP80-DexOx-AmB nanoparticle conjugates have 2.8 µg and 9.7 µg of immobilized AmB respectively, and therefore the MIC values are above the MIC of soluble AmB (0.9 ± 0.2 µg/ml). Nevertheless, growth assays performed over 15 hours and illustrated in Figure 7 showed that 5 µg/ml of SNP5-DexOx-AmB15% or 50 µg/ml of SNP80-DexOx-AmB15% are fungistatic, i.e. arrest the growth of *Candida albicans.*

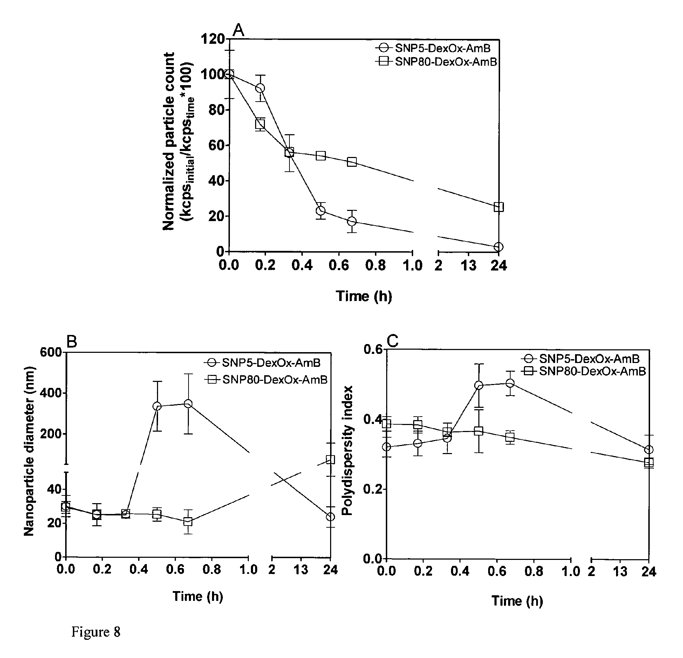

The lower MIC value observed for AmB immobilized into the nanoparticles compared with its soluble formulation might be explained by the aggregation of the nanoparticles in YPD. Accordingly, the stability of nanoparticles functionalized with DexOx-AmB when suspended in YPD medium at a concentration of 0.3 mg/ml was investigated. The results of these experiments are illustrated in Figure 8. Without agitation, nanoparticles tend to increase in size and sediment over time. The sedimentation rate appears to increase with decreasing particle size.

### 6.4 Spectrum of antifungal activity

In order to determine the spectrum of antifungal activity of the nanoparticles functionalized with amphotericin B, they were tested against four strains of *Candida,* namely: C *parapsilosis,* C *krusei,* C *glabrata,* and C *tropicalis.* The MIC values were determined after 8 hours of incubation with AmB, DexOx-AmB conjugate, unconjugated silica nanoparticles, and silica nanoparticles functionalized with DexOx-AmB. The results are illustrated in Table 7 below:

**Table 7: Antifungal activity of AmB, SNPs without AmB conjugate, DexOx-AmB conjugate, and SNPs conjugated with AmB.**

| **Formulation** | **Minimum Inhibitory Concentration (MIC)*** | | | | |
|---|---|---|---|---|---|
| | ***C*. *albicans*** | ***C. krusei*** | ***C*. *parapsilosis*** | ***C. glabrata*** | ***C. tropicalis*** |
| AmB | 0.3 | 0.1 | 0.3 | 0.03 | 0.08 |
| DexOx23AmB15 | 0.5(0.2) | 1(0.5) | >8(>3.7) | 0.2(0.09) | 1(0.5) |
| SNP5 | >5,000 | - | - | - | - |
| SNP5NH₂(12mg/ml) DexOx23AmB15 | 100(1) | 1,000(10) | 1000-2000(10-20) | 300(3) | 100(1) |

| | | | | | |
|---|---|---|---|---|---|
| The values in parentheses refer to the amount of AmB in the conjugates or nanoparticles. *The MIC corresponds to the lowest concentration of the compound that inhibited visible growth of the microorganism. Data corresponds to the average of three independent measurements. | | | | | |

These results indicate that the *Candida* strains are susceptible to AmB to different extents, with *C*. *glabrata* being the most susceptible. The DexOxAmB conjugate was very effective for *C*. *albicans* but not for *C*. *parapsilosis.* As expected, unconjugated silica nanoparticles were ineffective in killing *Candida* species; however nanoparticles functionalized with AmB were very fungicidal. The killing efficiency of the nanoparticles was 3 to 33-fold lower than the one observed for free AmB. Several reasons might explain the decrease in the activity of the NPs including (i) their aggregation (see above), (ii) the inappropriate orientation of AmB immobilized at the surface of the NP, and (iii) the excessive number of attachment points of DexOxAmB into the surface of SNP reducing the overall mobility of AmB.

### Example 7: Comparison with Silver nanoparticles

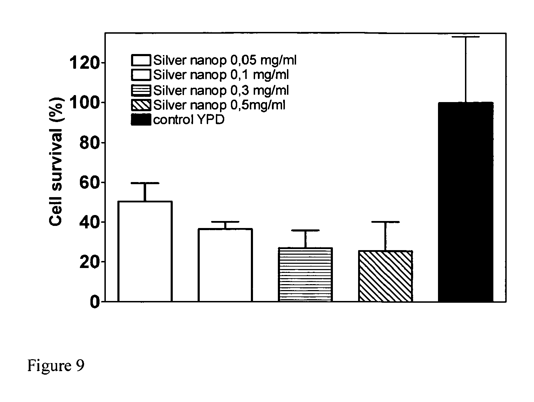

Recent studies indicate that silver nanoparticles can be used as antifungal agents (Panacek et al Biomaterials 2009, 30(31) 6333-6340; Kim et al., J Microbiol and Biotech 2008, 18(8), 1482-1484). Silver nanoparticles have shown fungicidal activity against *C*. *albicans* at the concentration of 27 µg/ml (Panacek et al. Biomaterials 2009, 30(31) 6333-6340). Therefore the antifungal activity of silver nanoparticles was compared with that of silica nanoparticles using the methodology described above. It was observed that suspensions of silver nanoparticles up to 500 µg/ml were ineffective in killing all the fungi (Figure 9). In addition, silver nanoparticles are not fungistatic at the concentrations observed for SNP5 and SNP80 nanoparticles incorporating AmB. Therefore, under the conditions tested, the silica nanoparticles functionalized with AmB are surprisingly more effective against *C*. *albicans* than silver nanoparticles. This is particularly advantageous since despite the inherent antifungal properties of silver, it is cytotoxic to mammalian cells (Zhan et al., Anal. Chem. 2007, 79, 5225; Poon et al., Burns 2004, 30, 140) and silver resistance has been documented in bacteria strains isolated from hospitals (Silver et al., FEMS Microbiology Reviews 2003, 27, 341). Thus, silica nanoparticles provide a viable alternative to the use of silver in antimicrobial coatings.

### Example 8: Immobilization of SNP-DexOx-AmB conjugate on a surface

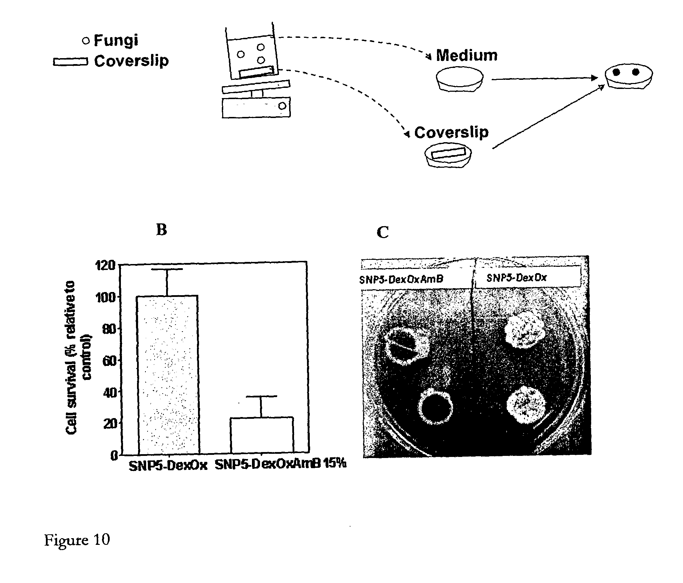

In order to evaluate the use of the SNP-DexOx-AmB conjugates for the preparation of antifungal coatings, the conjugates were immobilized on a glass surface. The immobilization was performed using a methodology described by Lee et al., Adv. Mater Deerfield 2009, 21(4), 431-434, using polydopamine. A schematic representation of the assay is shown in Figure 10. Briefly, dopamine was first polymerized on top of a coverslip and then the reactivity of the polydopamine was used against the remaining amine groups present on SNP5-NH₂-DexOxAmB15%. Using this technique, nanoparticles with a total amount of 3.1 µg AmB were immobilized at the surface of each coverslip (1.13 cm²). In order to confirm that the nanoparticles were permanently attached to the surface, the coverslip was washed in YPD medium for 2 hours under agitation (200 rpm), and the washing medium tested against *Candida.* No measurable antifungal activity was detected.

The coverslips were then incubated for 2 hours with 1×10³ cells of *C*. *albicans* to assess their antifungal activity. A 78% reduction in fungi was observed in the media containing the coverslip coated with SNP functionalized with AmB relative to the control (coverslip coated with SNP5-NH₂-DexOx). Finally, the culture medium was removed and the coverslips were rinsed twice with sterile water (1 ml) to remove non-adherent cells and plated upside down on YPD agar. After 72 hours, no fungal colonies were observed on the coverslips coated with SNP5-NH₂-DexOxAmB15% whereas fungi colonized the control coverslips coated with SNP5-NH₂-DexOx.

### Example 9: Hemolysis Assay

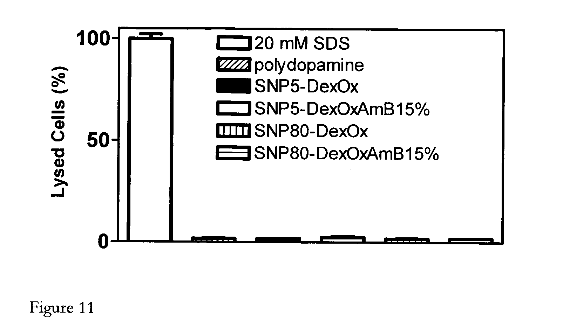

In order to assess the hemocompatibility of a surface coated with the SNP5-DexOx-AmB and SNP80-DexOx-AmB conjugates, coated coverslips were prepared as described above (example 7) and samples were incubated with red blood cells as illustrated in Table 8 below. Whole blood with anticoagulant (EDTA) was centrifuged for 10 minutes at 600 xg at 4°C. The pellet was resuspended in PBS 7.2. The red blood cells were then diluted in PBS to obtain a final concentration of 2 x 10⁸ cells/0.9 ml. in a red blood cell suspension (RBC suspension). The RBC suspension was then incubated with 0.1 ml PBS 7.2 and the coated coverslips for 1 hour at 37°C and with shaking at 90 rpm. Cell suspensions were then centrifuged at 600 xg and 4°C, and the absorbance of the supernatant at 540 nm was measured. A control sample, in which the RBC suspension was incubated with SDS (20mM) to induce significant lysis, was also measured. UV-Vis absorbance measurements are illustrated in Figure 11.

**Table 8: Samples for hemocompatability assessment**

| Sample No. | Sample (0.9ml RBC suspension with 0.1 ml PBS 7.2 plus..) |
|---|---|
| 1 | Coverslip coated with SNP5-DexOx-AmB |
| 2 | Coverslip coated with SNP5-DexOx |
| 3 | Coverslip coated with SNP80-DexOx-AmB |
| 4 | Coverslip coated with SNP80-DexOx |
| 5 | Coverslip coated with polydopamine |
| 6 | - |

From Figure 11 it is immediately apparent that the coated coverslips did not cause lysis of the red blood cells, indicating that the SNP-DexOx-AmB conjugates are hemocompatible.

### Example 9: Cytotoxicity Assay

In order to assess the cytocompatibility of a surface coated with the SNP5-DexOx-AmB and SNP80-DexOx-AmB conjugates, coated coverslips were prepared as described above (example 7) and samples were incubated with mononuclear cells. Mononuclear cells were obtained from single or pooled umbilical cord blood samples after Ficoll (Histopaque-1077 Hybri Max; Sigma-Aldrich, St. Louis, USA) density gradient separation. Coverslips were initially washed with 0.5 ml EGM-2 (Lonza) having 0.5% (v/v) PenStrep solution (5h at 37°C). The solution was then removed and 1 ml of EGM-2 containing 5 x 10⁵ mononuclear cells added. After 24 hours of incubation, a MTT test was performed (as described in Mosmann T. Journal of Immunological Methods 1983, 65, 55) and the formazan crystals formed were quantified by measuring the absorbance at 540 nm.

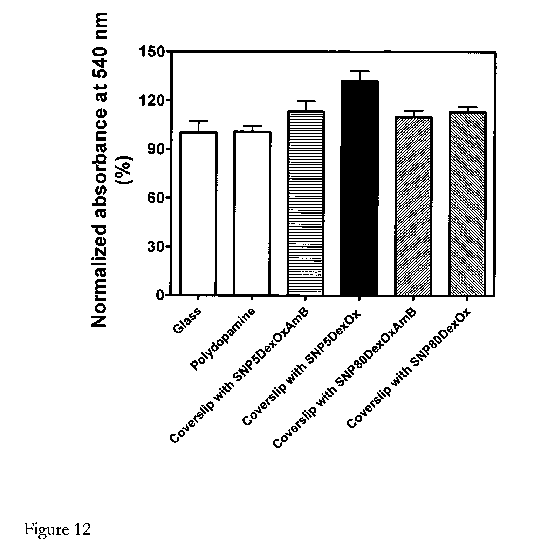

From Figure 12, it can be seen that no measurable effect in the cellular metabolism was observed indicating that the coatings were substantially non-cytotoxic.

It is apparent that the antimicrobial nanoparticle conjugates of the invention demonstrate effective antimicrobial activity both in suspension and when immobilized on surfaces. In addition, the conjugates are hemocompatible and substantially non-cytotoxic. Thus, the conjugates of the invention can be used to create highly active and long-lasting antimicrobial coatings for objects such as medical devices. Nanoparticles allow for efficient immobilization of the antimicrobial conjugates to ensure leaching of the antimicrobial agent is minimized and a long-lasting antimicrobial effect can be achieved. In particular embodiments of the invention, silica nanoparticle-antifungal conjugates demonstrate surprisingly superior efficacy in the treatment of several strains of fungi, when compared with known formulations.

Thus, the inventive antimicrobial nanoparticle conjugates act as efficient antimicrobial agents and can be used either in suspension or immobilised onto surfaces, such as the surfaces of medical devices to provide effective, broad-spectrum antimicrobial coatings.

The method also encompasses novel methods of conjugating the antimicrobial agent to the silica nanoparticle by a linker molecule. The method allows direct conjugation of the antimicrobial agentto the surface of the nanoparticle without requiring the use of a hydrogel, or amphogel, as in previous reported methodologies. The efficient presentation of the antimicrobial moiety on the nanoparticle surface ensures that the antimicrobial properties of the agent can be maintained during the conjugation process so that efficient antimicrobial activity can be demonstrated for the resultant conjugate. In addition, leaking of the antimicrobial agent from the conjugate can be minimised, ensuring that antimicrobial activity is mediated primarily by contact with the antimicrobial agent, and allowing antimicrobial surfaces to be reused without appreciable loss of antimicrobial activity.

Detailed description of the drawings:
Figure 1: A) SEM image of silica nanoparticles with a diameter of 170 nm; B) SEM image of silica nanoparticles with a diameter of 15 nm and C) TEM image of silica nanoparticles with a diameter of 5 nm.
Figure 2: UV-VIS spectra of DexOx, AmB and DexOxAmB10% conjugate dissolved in water (DexOx and DexOxAmB at concentrations of 8.8 and 13.5 µg/ml, respectively) or in DMSO (AmB, at concentration of 4.8 µg/ml). The UV-VIS absorption spectrum of AmB spectrum shows three peaks with high intensity at 416, 391 and 371 nm and two peaks with small intensity at 350 and 330 nm. As expected, the spectrum of the conjugate shows the same peaks.
Figure 3: ¹H NMR spectra of (A) AmB, (B) DexOx-23 (the number of oxidized residues per 100 glucose residues is 23), and (C) DexOx-23AmB15% (the number of AmB residues per 100 glucose residues is 15). Spectra of AmB and DexOxAmB were obtained in DMSO-d6 while DexOx-23 was obtained in D₂O both at 25°C. The anomeric proton of glucose in dextran is denoted as 1. The methyl groups of AmB are denoted as Me.
Figure 4: Size distribution by number of SNPs functionalized with DexOxAmB (A: 170 nm; B: 80 nm; C: 5 nm). Nanoparticles were dispersed in distilled water and sonicated before measurements. The results show that most of the SNP170 nanoparticles have diameters around 135 nm and a small population with 50 nm. SNP80 nanoparticles have a unimodal distribution around 330 nm, while SNP5 nanoparticles have a unimodal distribution around 22 nm.
Figure 5: Yeast killing by SNPs containing DexOx-AmB.
   A) Antifungal activity of nanoparticles with different diameters: SNP170, SNP80 and SNP5. Unconjugated nanoparticles (SNP170-NH₂ and SNP80-NH₂) have minimal antifungal activity against *Candida albicans.* In contrast, nanoparticles initially functionalized with amine groups and then reacted with variable concentrations of DexOx-AmB conjugate (1.5, 6.0 or 12.0 mg/ml) having 10% of AmB (these percentages indicate the amount of AmB per 100 dextran glucopyranoside residues) killed all the micro-organisms. The antifungal activity of SNP170-NH₂-DexOx-AmB nanoparticles slightly decreases if they are reused. This contrasts with the activity profile of SNP15 and SNP5-NH₂-AmB nanoparticles showing no apparent reduction in their antifungal activity after 5 reuses.
   B) Antifungal activity of nanoparticles having different amounts of conjugated AmB. The amount of AmB per mg of nanoparticle varied from 12.1 µg to 38 µg per mg of nanoparticles. In A and B, the results are expressed as the mean ± SD.
Figure 6: Nanoparticles functionalized with AmB kill *Candida* mainly by contact. The nanoparticles were washed for 8 hours in YPD, the supernatant collected and tested against *Candida.* Cell survival was higher than 60%; however if the effect of the supernatant of unconjugated nanoparticles is subtracted than cell survival is higher than 80%. Next, the nanoparticles were tested against *Candida* killing all the microorganisms. The nanoparticles were centrifuged and the supernatant collected and exposed to *Candida.* More than 70% of the cells survived.
Figure 7: Yeast growth kinetics in the presence of nanoparticles. The results are expressed as the mean ± SD (*n*=3). A suspension of nanoparticles with or without AmB, at the concentrations presented in the figure, were incubated with 1 × 10⁵ cells/ml of *Candida albicans* in YPD medium over 15 h and the absorbance of the medium measured at 600 nm.
Figure 8: Stability of nanoparticle suspension over time. SNP80-NH₂-DexOxAmB and SNP5-NH₂-DexOxAmB nanoparticle suspensions (0.3 mg/ml in YPD) were measured in a Brookhaven apparatus overtime without agitation. A) Normalized particle count. The particle counts (kcps) at the end of each measurement were normalized relatively to the initial value. B) Nanoparticle diameter. C) Polydispersity index.
Figure 9: Yeast killing by silver nanoparticles. The results are expressed as mean ± SD. A suspension of silver nanoparticles (0.1 mg/ml; average diameter of nanoparticles: 10 nm) was centrifuged and then re-suspended in YPD medium at the concentrations presented in the figure. The activity was measured according to the protocol defined in the Materials and Methods, for 8 h using 1 × 10⁵ cells as inoculum.
Figure 10: Yeast killing by SNP5 functionalized with AmB immobilized on a surface.
   A) Schematic representation of the bioactivity assay. SNP5-NH₂-DexOx-AmB or SNP5-NH₂-DexOx coated coverslips were incubated in 1 ml liquid YPD medium containing 1 x 10³ *Candida albicans* for 2 hours under agitation. Then, aliquots of the medium and the coverslips were plated on YPD agar and the plates were incubated for 72 h for CFU count.
   B) Antifungal activity of immobilized SNP5-NH₂-DexOx-AmB on planktonic *Candida albicans* cells. The CFU counts are normalized relative to the control (SNP5-NH₂-DexOx) and expressed as mean ± SD (n=4).
   C) Photograph of coverslips coated with SNP5-DexOx-AmB (left) and SNP5-DexOx (right) plated on YPD agar after incubation in medium with *Candida albicans.*
Figure 11: Results of the hemolysis assay. Glass coverslips were coated with polydopamine and then reacted with SNP5-DexOx, SNP5-DexOx-AmB15%, SNP80-DexOx, SNP80-DexOx-AmB15%, according to the protocol defined in Example 7. These coverslips were then incubated with 2 x 10⁸ red blood cells in 1 ml for 1 hour at 37°C with shaking at 90 rpm. Cell suspensions were then centrifuged at 600 xg and 4°C, and the absorbance of the supernatant at 540 nm was measured. A control sample, in which the red blood cell suspension was incubated with SDS (20mM) to induce significant lysis, was also measured. The values of Abs 540 nm were normalized relatively to the control (100% lysis, SDS 20mM) (average ± S.D., *n*=3).
Figure 12: Mitochondrial metabolic activity of mononuclear cells plated on top of coverslips with different coatings. Results are average ± SD, n=6. Each absorbance at 540 nm was normalized by the one obtained on glass coverslip.

## Claims

1. An antimicrobial nanoparicle conjugate comprising an antimicrobial agent immobilized to a nanoparticle by a linker molecule.

2. An antimicrobial nanoparticle conjugate as claimed in claim 1, wherein the antimicrobial agent is covalently immobilized to the nanoparticle by the linker molecule.

3. An antimicrobial nanoparticle conjugate as claimed in any preceding claim wherein the linker molecule comprises an activated polymer.

4. An antimicrobial nanoparticle conjugate as claimed in claim 3, wherein the concentration of the antimicrobial agent present on the surface of the nanoparticle is a function of the degree of activation of the polymer.

5. An antimicrobial nanoparticle conjugate as claimed in claim 3 or claim 4, wherein the polymer is a polysaccharide.

6. An antimicrobial nanoparticle conjugate as claimed in any preceding claim, wherein the nanoparticle is a silica nanoparticle.

7. An antimicrobial nanoparticle conjugate as claimed in any preceding claim, wherein the antimicrobial agent is an antibiotic, antifungal or antibacterial.

8. A process of preparing an antimicrobial nanoparticle conjugate as claimed in any of claims 1 to 7 comprising the steps of:
providing an activated-linker-antimicrobial conjugate; and
coupling the conjugate to a nanoparticle.

9. A method of immobilizing an antimicrobial agent to a nanoparticle comprising the steps of:
functionalizing the nanoparticle;
preparing an activated linker;
reacting the activated linker with the antimicrobial agent to form an activated-linker-antimicrobial conjugate;
and
reacting the activated-linker-antimicrobial conjugate with the functionalized nanoparticle to form an antimicrobial-functionalized nanoparticle.

10. A method of immobilizing an antimicrobial agent to a nanoparticle as claimed in claim 9, further comprising the step of:
reducing the antimicrobial-functionalized nanoparticle to form a stable antimicrobial nanoparticle conjugate.

11. A method of immobilizing an antimicrobial agent to a nanoparticle as claimed in claim 10, wherein at least one of the steps of reacting the activated linker with the antimicrobial agent and reacting the activated-linker-antimicrobial conjugate with the functionalized nanoparticle is carried out in aqueous solution.

12. Use of an antimicrobial nanoparticle conjugate as claimed in any of claims 1 to 7 as an antimicrobial coating for a medical device or surgical material.

13. A method of preparing a medical device or surgical material with an antimicrobial coating, wherein the method comprises coating at least one surface or part of a surface of the device or material with an antimicrobial nanoparticle conjugate as claimed in any of claims 1 to 7.

14. A method of preparing a medical device or surgical material with an antimicrobial coating as claimed in claim 13, wherein the at least one surface or part of a surface is treated with an adhesive prior to coating.

15. A medical device or surgical material with an antimicrobial coating, obtainable by the method of claim 13 or claim 14.

16. A medical, dental or surgical device or material comprising at least one surface or part of a surface coated with, or formed from a material containing, an antimicrobial nanoparticle conjugate as claimed in any of claims 1 to 7.

17. A medical, dental or surgical device or material as claimed in claim 16, wherein the surface or part of a surface has antimicrobial activity at or below 10 µg/cm² of the antimicrobial.
